# EUROPEAN PATENT SPECIFICATION

(11) **EP 3 027 215 B1**
(45) Date of publication and mention of the grant of the patent: **03.05.2017**
(21) Application number: 14736479.8
(22) Date of filing: 18.04.2014
(51) Int. Cl.: A61K 47/12, A61K 9/08, A61K 9/00, A61K 31/522

(54) **POWDER FORMULATION OF VALGANCICLOVIR**
PULVERFORMULIERUNG AUS VALGANCICLOVIR
FORMULATION EN POUDRE DE VALGANCICLOVIR

(30) Priority: 02.08.2013 TR 201309416
(43) Date of publication of application: 08.06.2016
(73) Proprietor: Ali Raif Ilaç Sanayi ve Ticaret Anonim Sirketi, 34418 Istanbul (TR)
(72) Inventor: KESGIN, Didehan, Esenyurt / Istanbul (TR)
(74) Representative: Bulut, Pinar
(86) International application number: PCT/TR2014/000124
(87) International publication number: WO 2015/016789

(56) References cited:
- WO-A2-2008/071573
- STEFANIDIS DIMITRIOS ET AL: "Reactivity of valganciclovir in aqueous solution", DRUG DEVELOPMENT AND INDUSTRIAL PHARMACY, NEW YORK, NY, US, vol. 31, no. 9, 1 October 2005 (2005-10-01), pages 879-884, XP009105970, ISSN: 0363-9045, DOI: 10.1080/03639040500271951
- HENKIN CAROLYN C ET AL: "Stability of valganciclovir in extemporaneously compounded liquid formulations", AMERICAN JOURNAL OF HEALTH-SYSTEM PHARMACY, AMERICAN SOCIETY OF HEALTH-SYSTEM PHARMACISTS, US, vol. 60, no. 7, 1 April 2003 (2003-04-01), pages 687-690, XP009105971, ISSN: 1079-2082

## Description

### Technical Field:

The present invention relates to the powder formulation of valganciclovir hydrochloride for oral administration after being reconstituted in water.

### Prior Art:

Valganciclovir Hydrochloride is a strong antiviral agent which is used in the treatment of cytomegalovirus (CMV) retinitis in the patients with acquired immunodeficiency syndrome (AIDS) and in the prevention of cytomegalovirus (CMV) disease in patients with solid organ transplantation.

Valganciclovir is a prodrug which is L-valyl ester of ganciclovir,. It exists as the mixture of two diastereoisomers. When administered orally, both of these diastereoisomers rapidly turn into ganciclovir. Ganciclovir is a synthetic analogue of guanine.

The advantage of valganciclovir is that it shows a similar effect *in vivo* to that of the ganciclovir given by intravenous route, while it is more effective than ganciclovir capsules when it comes to oral administration. Ganciclovir was approved by FDA in March 2001 for use in the induction of CMV retinitis treatment, in maintenance treatment following the induction, or for use in the patients with inactive CMV retinitis. Furthermore, the use of Valganciclovir for CMV prophylaxis after heart, kidney or kidney-pancreas transplantation was approved.

The chemical name of valganciclovir hydrochloride is L-Valine, 2-[(2-amino-1,6-dihydro-6-oxo-9H-purin-9-yl)methoxy]-3-hydroxypropyl ester, monohydrochloride. (I)

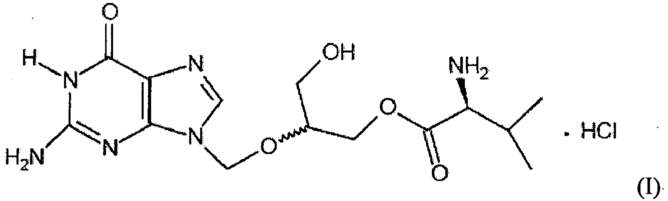

Valganciclovir hydrochloride is commercially available under the trade name Valcyte® in tablet form or powder form for oral solution.

Molecular formula of Valganciclovir Hydrochloride is C₄H₂₂N₆O₅.HCl, while the molecular weight of the same is 390.83. Valganciclovir hydrochloride is a polar hydrophilic compound with a solubility of 70 mg/ml in water at 25°C at pH 7.0 and n-octanol-water partition coefficient of 0.0095 at pH 7.0. The pKa of valganciclovir hydrochloride is 7.6. Valganciclovir hydrochloride is freely soluble under acidic conditions with a maximum solubility higher than 200 mg/ml at a pH in the range of 4-6. Valganciclovir hydrochloride has maximum stability at a pH below 3.8.

Valganciclovir hydrochloride, in the solid form, shows acceptable physical, chemical, and light stability when stored under room temperature conditions. No special storage conditions are required except that excessive humidity must be avoided.

In the state of the art, PCT application No. WO2008/071573 (F.Hohhmann-LA Roche AG) regarding powder formulation of valganciclovir relates to a solid pharmaceutical dosage form for oral administration, after being constituted in water said formulation comprising therapeutically efficient amount of valganciclovir hydrochloride and fumaric acid which is a non-hygroscopic organic acid.

The use of citric acid or potassium acetate in acidic solutions in order to stabilize valganciclovir is disclosed (Stefanidis Dimitrios et al., "Reactivity of valganciclovir in aqueous solution" Drug Development and Industrial Pharmacy October 2005, Volume 31, no.9, pages 879-884, XP009105970 ISSN: 0363-9045).

Stability of valganciclovir in aqueous preparations under acidic conditions is disclosed (Henkin Carolyn C. et al., "Stability of valganciclovir in extemporaneously compounded liquid formulations" American Journal of Health-system Pharmacists April 1, 2003, Volume 60, no.7, April 1, 2003, pages 687-690, XP009105971 ISSN: 1079-2082). Short-term stability data has shown that liquid dosage forms are unstable for the anticipated shelf life of the product.

Therefore, efforts focused on powder dosage forms for later constitution with water, to provide a reasonable shelf life for the valganciclovir hydrochloride, and thus the liquid dosage form to be prepared therefrom.

### Description of the Invention:

The present invention relates to the powder formulation of valganciclovir hydrochloride for oral administration after being reconstituted in water.

The present invention relates to the use of an organic acid in the formulation in order to increase the stability of the valganciclovir and to make the shelf life of the liquid dosage form prepared from the solid powder longer since valganciclovir hydrochloride is very soluble under acidic conditions. Tartaric acid is selected as the organic acid; wherein it lowers the pH of the solution to be prepared below 3.5, approximately to pH 3.0. The compound which is used is L-tartaric acid, which complies with European Pharmacopoeia specifications.

The present invention relates to the selection of the most appropriate formulation by analyzing the effect of the organic acid used in the formulation on degradation products of valganciclovir.

Test formulations are given in Table 1.

**Table 1. Test formulations, Formula 1**

| | **mg/120 mg** | **g/bottle** | **Prepared Solution, mg/ml** |
|---|---|---|---|
| Valganciclovir HCl | 55.15 | 5.515 | 55.15 |
| Povidone K-30 | 2.0 | 0.2 | 2.0 |
| L-Tartaric Acid | 2.0 | 0.2 | 2.0 |
| Sodium Benzoate | 1.0 | 0.1 | 1.0 |
| Sodium Saccharin | 0.25 | 0.025 | 0.25 |
| Mannitol | 57.8 | 5.78 | 57.8 |
| Tutti Frutti Flavor | 1.8 | 0.18 | 1.8 |
| Purified water | q.s. | q.s. | 0.91 ml |
| **Total** | 120 mg | 12 g | 1.0 ml |

**Table 2. Test formulations, Formula 2**

| | **mg/120 mg** | **g/bottle** | **Prepared Solution, mg/ml** |
|---|---|---|---|
| Valganciclovir HCl | 55.15 | 5.515 | 55.15 |
| Povidone K-30 | 2.0 | 0.2 | 2.0 |
| Fumaric Acid | 2.0 | 0.2 | 2.0 |
| Sodium Bezoate | 1.0 | 0.1 | 1.0 |
| Sodium Saccharin | 0.25 | 0.025 | 0.25 |
| Mannitol | 57.8 | 5.78 | 57.8 |
| Tutti Frutti Flavor | 1.8 | 0.18 | 1.8 |
| Purified water | q.s. | q.s. | 0.91 ml |
| **Total** | 120 mg | 12g | 1.0 ml |

Wet granulation is used as the production process.

In the present invention, the powder formulation comprises sodium benzoate as preservative agent, sodium saccharin as sweetener, mannitol as bulking agent, tutti frutti as flavoring agent, and povidone K-30 as binder.

Stability study has been carried out for both of prepared formulations; and impurity results are analyzed in Table 3 and Table 4.

**Table 3.**

| Comparison of the stability data of the powder prepared for the oral solution | | | | |
|---|---|---|---|---|
| Formulation | Formula 1 | | Formula 2 | |
| Storage Conditions | Quantitation % Valganciclovir | Total Impurity | Quantitation % Valganciclovir | Total Impurity |
| Starting | 99.4% | 0.63% | 99.2% | 1.45% |
| 3 months 25°C / 60% RH | 99.5% | 0.64% | 99.0% | 1.53% |
| 6 months 25°C / 60% RH | 99.3% | 0.65% | 98.5% | 1.57% |
| 3 months 30°C / 65% RH | 99.2% | 0.66% | 98.6% | 1.67% |
| 6 months 30°C / 65% RH | 98.8% | 0.69% | 98.3% | 1.73% |

**Table 4.**

| Comparison of the stability data of the solutions have been prepared | | | | |
|---|---|---|---|---|
| Formulation | Formula 1 | | Formula 2 | |
| Storage Conditions | Quantitation % Valganciclovir | Total Impurity | Quantitation % Valganciclovir | Total Impurity |
| Starting | 100.7% | 0.71% | 102.1% | 1.75% |
| 1 month 5°C | 100.5% | 0.75% | 101.6% | 1.88% |
| 2 months 5°C | 100.4% | 0.78% | 99.4% | 1.94% |
| 3 months 5°C | 100.4% | 0.82% | 98.6% | 2.14% |

As seen in the table, Formula 1 (the formulation comprising tartaric acid) has yielded better impurity results when compared to the Formula 2 (the formulation comprising fumaric acid).

## Claims

1. The powder formulation comprising valganciclovir hydrochloride for oral administration after being reconstituted in water, **characterized in that** it comprises L-tartaric acid as stabilizer.

2. The powder formulation comprising valganciclovir hydrochloride according to Claim 1, **characterized in that** the pH of the solution is below 3.5.

3. The powder formulation comprising valganciclovir hydrochloride according to Claim 1, **characterized in that** it is prepared by wet granulation.

4. The powder formulation comprising valganciclovir hydrochloride according to Claims 1 to 3, **characterized in that** it comprises sodium benzoate as the preservative agent.

5. The powder formulation comprising valganciclovir hydrochloride according to Claims 1 to 3, **characterized in that** it comprises sodium saccharin as the sweetener.

6. The powder formulation comprising valganciclovir hydrochloride according to Claims 1 to 3, **characterized in that** it comprises mannitol as the bulking agent.

## Patentansprüche

1. Pulverisierte Formulation umfassend Valganciclovir-Hydrochlorid zur oralen Einnahme nach dem Auflösen im Wasser **gekennzeichnet durch** das Beinhalten der L-Weinsäure als Stabilisierungsmittel.

2. Pulverisierte Formulation umfassend Valganciclovir-Hydrochlorid nach Anspruch 1 **gekennzeichnet durch** das Liegen des pH Werts der Auflösung unter 3,5.

3. Pulverisierte Formulation umfassend Valganciclovir-Hydrochlorid nach Anspruch 1 **gekennzeichnet durch** das Vorbereiten in der feuchten Granulierung.

4. Pulverisierte Formulation umfassend Valganciclovir-Hydrochlorid nach einem der Ansprüche 1 bis 3 **gekennzeichnet durch** das Beinhalten Natriumbenzoat als Schutzmittel.

5. Pulverisierte Formulation umfassend Valganciclovir-Hydrochlorid nach einem der Ansprüche 1 bis 3 **gekennzeichnet durch** das Beinhalten Natriumsaccharin als Süßstoff.

6. Pulverisierte Formulation umfassend Valganciclovir-Hydrochlorid nach einem der Ansprüche 1 bis 3 **gekennzeichnet durch** das Beinhalten Mannitol als Massenbildner.

## Revendications

1. Formulation en poudre comprenant du Chlorhydrate de Valganciclovir pour administration orale après reconstitution dans l'eau, **caractérisée en ce qu'**elle comprend de l'acide L-tartrique comme stabilisant.

2. Formulation en poudre comprenant du Chlorhydrate de Valganciclovir selon la revendication 1, **caractérisée en ce que** le pH de la solution est inférieur à 3,5.

3. Formulation en poudre comprenant du Chlorhydrate de Valganciclovir selon la revendication 1, **caractérisée en ce qu'**elle est préparée par granulation humide.

4. Formulation en poudre comprenant du Chlorhydrate de Valganciclovir selon les revendications 1 à 3, **caractérisée en ce qu'**elle comprend du benzoate de sodium comme agent conservateur

5. Formulation en poudre comprenant du Chlorhydrate de Valganciclovir selon les revendications 1 à 3, **caractérisée en ce qu'**elle comprend de la saccharine sodique comme édulcorant.

6. Formulation en poudre comprenant du Chlorhydrate de Valganciclovir selon les revendications 1 à 3, **caractérisée en ce qu'**elle comprend du mannitol comme agent gonflant.
